# FASCICULE DE BREVET EUROPEEN

(11) **EP 1 053 746 B1**
(45) Date de publication et mention de la délivrance du brevet: **27.11.2002**
(21) Numéro de dépôt: 00401344.7
(22) Date de dépôt: 17.05.2000
(51) Int. Cl.: A61K 9/16, A61P 35/00

(54) **Utilisation de microsphères biodégradables libérant un agent anticancéreux pour le traitement du glioblastome**
Verwendung von biologisch abbaubaren Mikrosphären zur Freisetzung eines Krebsmittels zur Behandlung des Glioblastoms
Use of biodegradable microspheres for the delivery of a anticancer agent for the treatment of glioblastoma

(30) Priorité: 17.05.1999 FR 9906207
(43) Date de publication de la demande: 22.11.2000
(60) Demande divisionnaire: 02008921.5
(73) Titulaire: Laboratoires des Produits Ethiques Ethypharm, 78550 Houdan (FR)
(72) Inventeur: Faisant, Nathalie, 49220 Montreuil sur Maine (FR); Benoit, Jean-Pierre, 49240 Avrille (FR); Menei, Philippe, 49240 Avrille (FR)
(74) Mandataire: Ahner, Francis

(56) Documents cités:
- FR-A- 2 693 905
- NN: "Proceedings 1997 of the 24th International Symposium on controlled release of bioactive materials" 1997 , CONTROLLED RELEASE SOCIETY, INC. , DEERFIELD (US) XP002129728 * page 995 - page 996 * * référence 4 *
- T. PAINBENI ET AL.: "internal morphology of poly(D,L-lactide-co-glycolide) BNCU-loaded microspheres. Influence on drug stability" EUROPEAN JOURNAL OF PHARMACEUTICS AND BIOPHARMACEUTICS, vol. 45, no. 1, janvier 1998 (1998-01), pages 31-39, XP000727896 Amsterdam (NL)

## Description

La présente demande concerne l'utilisation de microsphères biodégradables libérant un anticancéreux, pour le traitement du glioblastome.

Le glioblastome appartient au groupe des maladies rares répertoriées par la National Organization for Rare Disorders.

Les tumeurs gliales malignes sont des tumeurs primitives du système nerveux central représentant, selon les séries, 13 à 22 % des tumeurs intracrâniennes. Sur le plan histologique, on distingue en fait deux types de tumeurs gliales malignes, les astrocytomes anaplasiques et les gliobastomes, ces derniers représentant la forme la plus indifférenciée de ces tumeurs.

Il n'existe actuellement pas de traitement efficace contre les tumeurs gliales malignes. La durée de survie des patients atteints de glioblastome ne dépasse pas un an, même si l'on associe la chimiothérapie et la radiothérapie à la chirurgie.

Le traitement des tumeurs gliales malignes est limité principalement par trois phénomènes.

Le premier est l'existence d'une barrière hémato-encéphalique (BHE) qui isole le système nerveux central du reste de l'organisme. Cette BHE ne laisse passer que des molécules de petite taille liposolubles. Les autres molécules doivent être administrées à des doses très importantes pour atteindre le système nerveux central et ce, au prix d'effets secondaires systémiques importants.

Le deuxième facteur limitant l'efficacité du traitement des tumeurs gliales est le caractère infiltrant de ces tumeurs. Le cerveau étant un organe hautement fonctionnel, il est impossible d'y réaliser une chirurgie large au sens carcinologique du terme. L'exérèse la plus complète possible ne sera qu'une exérèse macroscopiquement complète laissant un grand nombre de cellules tumorales infiltrées dans les parois de la cavité d'exérèse. De nombreux auteurs ont d'ailleurs montré que 90 % des tumeurs gliales malignes opérées et soignées par radiothérapie récidivaient dans les deux centimètres du site tumoral initial.

Le dernier facteur limitant l'efficacité du traitement des tumeurs gliales est le faible indice thérapeutique. Les cellules tumorales s'abritent en quelque sorte derrière un tissu normal extrêmement fragile et sensible aux agressions, provoquées par exemple par la radiothérapie ou par certains anticancéreux. Ainsi est-il difficile de détruire les cellules tumorales sans détruire les cellules nerveuses normales.

Les progrès réalisés dans le traitement des tumeurs gliales sont insuffisants (Kornblith PL, Walker M, Chemotherapy for malignant gliomas. J. Neurosurg, 68 : 1-17, 1988 ; Shapiro WR, Green SB, Burger PC, Selker RG, VanGilder JC, Robertson JT, Mahaley SM, A randomized comparaison of intra-arterial versus intravenous BCNU with or without intravenous 5-fluorouracil, for newly diagnosed patients with malignant glioma, J. Neurosurg. 76 : 772-781, 1992).

Actuellement, le traitement classique des gliobastomes consécutif à une résection chirurgicale est basé sur une radiothérapie externe. Il ne permet pas d'atteindre une durée de survie supérieure à un an. L'association d'une radiothérapie avec une chimiothérapie par la 1-(2-chloroéthyl)-3-cyclohexyl-1-nitroso-urée (BCNU) n'est efficace que sur les astrocytomes anaplasiques. Elle est d'un apport modeste car elle n'élève le pourcentage des survivants qu'à dix-huit mois, sans modifier la durée de survie.

Par ailleurs, l'immunothérapie ne s'est jamais imposée et la thérapie génique doit encore faire ses preuves.

Plusieurs techniques visant à augmenter la concentration locale d'anticancéreux ont été expérimentées, comme la rupture osmotique de la barrière hémato-encéphalique, l'injection dans le liquide céphalo-rachidien, la perfusion intra-carotidienne et l'administration intra-tumorale à l'aide de réservoirs sous-cutanés (Tamargo RJ et Brem H, Drug delivery to the central nervous system, Neurosurgery Quaterly, 2 : 259 - 279, 1992). Aucune de ces techniques n'a pu augmenter la durée de survie des patients et certaines se sont révélées hautement toxiques.

Au cours de ces dernières années, la recherche en pharmacie galénique a permis le développement de systèmes polymères implantables protégeant les substances actives d'une dégradation, et permettant leur libération locale de manière contrôlée pendant une période déterminée tout en diminuant les effets secondaires systémiques. Les avantages de ces systèmes polymères implantables ont récemment incité plusieurs équipes à étudier leur application dans les pathologies du système nerveux central (Langer R, Polymer implants for drug delivery in the brain, J. Controlled Release, 16: 53-60, 1991). En particulier, de tels systèmes implantés dans la paroi de résection tumorale de gliomes malins retardent la récidive tumorale et prolongent la survie des patients. Il persiste autour de la cavité opératoire des cellules malignes isolées, responsables de 90 % des récidives, qui se produisent dans les deux centimètres du foyer opératoire. Dans cette zone, le tissu nerveux est fonctionnel et la barrière hémato-encéphalique est encore intacte, ce qui limite l'action de la radiothérapie et de la chimiothérapie conventionnelle.

Divers systèmes polymères implantables libérant des molécules actives ont été développés et testés chez l'animal.

Un système de disques biodégradables composés de PCPP-SA (poly[1,3-bis(carboxyphenoxy)propane-co-sebacic acid]) et libérant du BCNU (GLIADEL®) a été développé malgré des résultats modestes en étude clinique (Brem H, Polymers to treat brain tumors, Biomaterials 11 : 699-701, 1990; Brem H, Mahaley MS, Vick NA, Black KL, Schold SC, Eller TW, Cozzens JW, Kenealy JN, Interstitial chemotherapy with drug polymer implants for the treatment of recurrent gliomas, J. Neurosurg 74 : 441-446, 1991 ; Brem H, Walter KA, Langer R, Polymers as controlled drug delivery devices for the treatment of malignant brain tumors, Eur J Pharm Biopharm, 39 (1) : 2-7, 1993 ; Brem H, Piantadosi S, Burger PC, Walker M, et al., Placebo-controlled trial of safety and efficacy of intraoperative controlled delivery by biodegradable polymers of chemotherapy for recurrent glioma, Lancet, 345 1008-1012, 1995).

Des microsphères libérant du BCNU ont été mises au point, mais les résultats des études chez l'animal étaient peu encourageants (Torres Al, Boisdron-Celle M, Benoit JP, Formulation of BCNU-loaded microspheres : influence of drug stability and solubility on the design of the microencapsulation procedure, J. Microencapsulation, 13 : 41-51, 1996 ; Painbéni T, Venier-Julienne MC, Benoit JP, Internal morphogogy of poly(D,L-lactide-co-glycolide) BCNU-loaded microspheres. Influence on drug stability, Eur. J. Pharm. Biopharm, 1998, 45, 31-39).

La publication de J.P. Benoît et al. parue dans « Proceedings 1997 of the 24^{th} International Symposium on controlled release of bioactive materials » décrit les résultats d'une étude clinique réalisée sur 8 patients auxquels on a administré des microsphères de 5-FU dans les parois du foyer opératoire après éxérèse de la tumeur. La durée de survie de chacun des 8 patients ayant participé à l'étude est de 80 semaines au maximum. Chez les deux patients ayant survécu à 17 et 20 mois, une récurrence locale de la tumeur a été observée.

L'objet de la présente invention est l'utilisation de microsphères biodégradables implantables libérant un anticancéreux, pour le traitement du glioblastome. L'utilisation de ces microsphères est associée à la radiothérapie et à la chirurgie. Après exérèse de la tumeur, les microsphères biodégradables qui libèrent un agent anticancéreux sont implantées dans le foyer opératoire par injection intratissulaire. Ensuite, on procède à une radiothérapie, au maximum dans les sept jours suivant l'intervention.

Grâce à l'utilisation de ces microsphères, la Demanderesse a réussi de façon tout à fait avantageuse à doubler la durée de survie des patients souffrant d'un glioblastome. En effet, l'utilisation des microsphères selon l'invention permet d'atteindre une durée de survie d'au moins 90 semaines.

En conséquence, la présente invention concerne l'utilisation de microsphères biodégradables libérant un agent anticancéreux radio-sensibilisant pour la fabrication d'un médicament destiné à être utilisé de façon simultanée, séparée ou étalée dans le temps avec une radiothérapie, pour le traitement du glioblastome, lesdites microsphères étant destinées à être implantées, après exérèse de la tumeur gliale, dans les parois du foyer opératoire, à au moins deux centimètres de profondeur, de préférence entre 2 et 3 centimètres, au moins tous les cm², les microsphères contenant l'agent anticancéreux étant enrobées d'un polymère qui retarde la libération de l'agent anticancéreux et maintient au cours du temps une concentration thérapeutiquement efficace dans l'espace parenchymateux en vue d'atteindre une durée de survie du patient ainsi traité au moins égale à environ 90 semaines, de préférence environ 130 semaines, de préférence encore environ 160 semaines.

Les microsphères utilisées dans le cadre de l'invention contiennent un anticancéreux qui est de préférence hydrophile et/ou ne franchit pas la barrière hémato-encéphalique. De façon avantageuse, l'agent anticancéreux ne présente pas une neurotoxicité centrale. Cet anticancéreux agit préférentiellement sur les cellules en division.

L'agent anticancéreux est constitué d'un composé anticancéreux radiosensibilisant ou d'un mélange de composés anticancéreux contenant au moins un composé anticancéreux radiosensibilisant, le ou lesdits composés anticancéreux étant par exemple choisis parmi le 5-fluorouracile (5-FU), les platines, comme le carboplatine et le cisplatine, les taxanes, comme le docétaxel et le paclitaxel, la gemcitabine, le VP16, la mitomycine, l'idoxuridine, les inhibiteurs de la topoisomérase 1, comme l'irinotécan, le topotécan et les camptothécines, les nitroso-urées, comme le BCNU, l'ACNU ou le MCNU, le méthotrexate, la bléomycine, l'adriamycine, le cytoxan et la vincristine, les cytokines immunomodulatrices, comme l'IL2, l'IL6, l'IL12 et l'IL13 et les interférons.

L'agent anticancéreux est de préférence le 5-FU.

Le 5-FU est un antimitotique ancien et bien connu. C'est une molécule hydrophile qui passe très faiblement la barrière hémato-encéphalique, son activité est donc augmentée par une administration locale (Bourke RS, West CR, Chheda G et al., Kinetics of entry and distribution of 5-fluorouracil in CSF and brain following intravenous injection in primate, Cancer Res, 33: 1735-1746, 1973; Gerosa MA, Dougherty DV, Wison CB, Rosenblum ML, Improved treatment of a brain tumor model, Part 2 : Sequential therapy with BCNU and 5-fluorouracil, J. Neurosurg. 58: 368, 1983; Kotsilimbas DG, Karpf R, Meredith S, Scheinberg LC, Evaluation of parenteral 5-FU on experimental brain tumors, Neurology, 16: 916-918, 1966; Levin VA, Edwards MS, Wara WM, Allen J, Ortega J, Vestnys P, 5-fluorouracil and 1-(2-chloroethyl)-3-cyclohexyl-1-nitrosourea (CCNU) followed by hydroxyurea, misonidazole and irradiation for brain stem gliomas : a pilot study of the brain tumor research center and the children cancer group, Neurosurgery, 14 : 679-681, 1984; Oda Y, Tokuriki y, Tsuda E, Handa H, Kieler J, Trial of anticancer pellet in malignant brain tumours, 5-FU and urokinase embedded in silastic. Proceeding of the 6^{th} European Congress of Neurosurgery, Acta neurochirurgica, Suppl. 28 : 489-490, 1979 ; Penn RD, Kroin JS, Harris JE, Chiu KM, Braun DP, Chronic intratumoral chemotherapy of a rat tumor with cisplatin and fluorouracil, Appl. Neurophysio, 46: 240-244, 1983; Shapiro WR, Studies on the chemotherapy of experimental brain tumors: Evaluation of 1-(2-Chloroethyl)-3-Cyclohexyl-1-Nitrosourea, Vinscristine and 5-Fluorouracil, J. Nat. Cancer Institute, 46(2), 359-368, 1971 ; Shapiro WR, Green SB, Burger PC, Selker RG, VanGilder JC, Robertson JT, Mahaley SM, A randomized comparison of intra-arterial versus intravenous BCNU with or without intravenous 5-fluorouracil, for newly diagnosed patients with malignant glioma, J. Neurosurg, 76 : 772-781, 1992 ; Soloway AH, Mark VH, Dukat EG et al., Chemotherapy of brain tumors. I-Transplanted murine ependymoblastomas, Cancer Chemother Rep., 36 : 1-4, 1964).

L'activité du 5-FU est aussi augmentée par une administration prolongée. Le 5-FU est un agent qui agit par interférence sur la synthèse des acides nucléiques. Des études ont montré que seulement 30 à 50 % des cellules d'un gliome malin murin (L9), et 14 à 44 % des cellules d'un gliome malin humain sont en division à tout moment. De plus, les durées du cycle cellulaire du glioblastome sont longues (20 heures pour le gliome L9, de 3 à 7 jours pour le glioblastome humain). Or, la clairance du 5-FU dans le plasma est rapide (demi-vie de 30 mn) (Neuwelt EA, Barnett PA, Frenkel EP, Chemotherapeutic agent permeability to normal brain and delivery to avian sarcoma virus-induced brain tumors in the rodent: observation on problems of drug delivery, Neurosurgery, 14: 154-160, 1984). Le 5-FU ne peut donc détruire, par une administration systémique ou une injection locale, un nombre important de cellules malignes.

Le 5-FU est essentiellement actif sur les tissus à renouvellement rapide et est exceptionnellement neurotoxique. Le 5-FU intervient dans la synthèse des acides nucléiques, dont les tissus à croissance rapide ont particulièrement besoin pour assurer leur prolifération et leur régénération. Ce n'est bien sûr pas le cas du tissu cérébral où les mitoses sont rares à l'état normal et ne surviennent qu'au sein de la population gliale. Les effets toxiques du 5-FU limitant son administration par voie générale sont essentiellement hématologiques et gastro-intestinaux. Si de rares effets secondaires neurologiques du 5-FU ont été publiés, leur étiopathogénie, peu connue, est probablement multifactorielle (blocage du cycle de Krebs par un catabolite du 5-FU ou une exacerbation d'un déficit en thiamine préexistant) (Aoki N, Reversible leukoencephalopathy caused by 5-fluorouracil derivatives, presenting as akinetic mutism, Surg Neurol, 25 : 279-282, 1986; Moore DH, Fowler WC, Crumpler LS, 5-fluorouracil neurotoxicity, case report, Gynecol Oncology, 36 : 152-154, 1990)

Enfin, le 5-FU est radio-sensibilisant (Koutcher JA, Alfieri AA, Thaler h et al., Radiation enhancement by biochemical modulation and 5-FU. Int. J. Radit. Biol. Phys., 39: 1145-1152, 1997). La supériorité de l'association 5-FU/radiothérapie dans chacun de ces traitements isolés a été mise en évidence dès les années 60 sur des modèles animaux et sur des cellules tumorales in vitro (Bagshaw M, A possible role of potentiation in radiation therapy, Amer J. Roentgenol, 85: 822-833, 1961 ; Vietti T, Eggerding F, Valeriote F, Combined effect of X-radiation and 5-fluorouracil on survival of transplanted leukemic cells, J. Natl. Inst., 47 : 865-870, 1971). Cette synergie serait due à la synchronisation de la population cellulaire tumorale et à la diminution des mécanismes de réparation cellulaire par le 5-FU. L'association radiothérapie et antipyrimidine (5-FU ou BrudR) a déjà été tentée chez l'homme (Goffman TE, Dachowski LJ, Bobo H et al., Long term follow-up on national cancer institute phase I/II study of glioblastoma multiforme treated with iododeoxyuridine and hyperfractionated irradiation, J Clinical Oncology, 10: 264-268, 1992). L'absence de franche efficacité peut encore ici s'expliquer par la voie d'administration systémique des drogues.

Lorsque l'agent anticancéreux est le 5-FU, la concentration en agent anticancéreux dans le liquide céphalo-rachidien, qui est le reflet de concentration dans l'espace parenchymateux, est comprise entre 3 et 20 ng/ ml.

Afin de limiter la neurotoxicité de l'agent anticancéreux contenu dans les microsphères utilisées dans le cadre de l'invention, on peut avantageusement adjoindre audit agent anticancéreux un composé neuroprotecteur. Ce composé neuroprotecteur est par exemple choisi parmi les facteurs de croissance peptidiques, comme le NGF ou le BDNF.

Les microsphères biodégradables utilisées dans le cadre de l'invention sont enrobées d'un polymère qui retarde la libération de l'agent anticancéreux et maintient, dans l'espace parenchymateux, une concentration thérapeutiquement efficace pendant un laps de temps d'au moins trois semaines, de préférence d'au moins quatre semaines.

Le polymère est choisi parmi l'éthylcellulose, le polystyrène, la poly(ε-caprolactone), le poly(d,l-acide lactique) et le poly(d,l-acide lactique-co-acide glycolique).

Le polymère est de préférence le poly(d,l-acide lactique-co-acide glycolique) ou PLAGA, qui est un polymère biodégradable autorisé dans la formulation de préparations galéniques à libération prolongée (à la différence du PCPP-SA qui n'est pas agréé pour une utilisation clinique de large envergure).

Le poly(d,l-acide lactique-co-acide glycolique) est de préférence du PLAGA 50: 50 (i.e. contenant une quantité égale d'acide lactique et d'acide glycolique), par exemple Resomer® RG 506 fourni par Bl Chimie, France, de masse moléculaire en masse égale à 72000, d'indice de polydispersité égal à 1,8 et de viscosité inhérente 0,80 dl/g (solution de polymère à 0,1 % dans le chloroforme à 25°C).

Le PLAGA est un copolymère hydrophobe dont la dégradation, provoquée par une réaction d'hydrolyse, donne naissance à deux substrats biologiques normaux, l'acide lactique et l'acide glycolique, métabolisés en fin de glycolyse aérobie en CO₂ et H₂O. Des études déjà anciennes ont montré que la voie respiratoire était la voie principale d'élimination de ces deux substrats. La vitesse de biodégradation du PLAGA dépend des proportions respectives d'acides lactique et glycolique. Le PLAGA est parfaitement biocompatible et provoque une réaction modérée aux corps étrangers (Visscher GE, RL Robinson, HV Mauding, Fong JW, Pearson JE, Argentieri GJ, Biodegradation of and tissue reaction to 50 : 50 poly(DL-lactide-co-glycolide) microcapsules, J. Biomed. Mat. Res. 19: 349-365, 1985). Le PLAGA entre dans la composition de fils chirurgicaux (Frazza EJ, Schmidt EE , A new absorbable suture, J. Biomed. Mater. Res., 5: 43-58, 1971) et de formes galéniques implantables en sous-cutané (Jalil R, Nixon JR, Biodegradable poly(lactic acid) and poly(lactide-co-glycolide) microcapsules: problems associated with preparative techniques and release properties (Review), J. Microencapsulation, 7: 297-325, 1990). Il a été démontré que les microsphères de PLAGA 50 : 50 peuvent être stérilisées par irradiation γ, et qu'une fois implantées par stéréotaxie dans le cerveau d'un rongeur, elles sont totalement biodégradées en deux mois, ne provoquant qu'une réaction modérée de type astrocytaire et histiocytaire non spécifique (Menei P, Daniel V, Montero-Menei C, Brouillard M, Pouplard-Barthelaix A, Benoit JP : Biodegradation and brain tissue reaction to poly(DL-lactide-co-glycolide) microspheres, Biomaterials 14 : 470-478, 1993 ; Menei P, Croue A, Daniel V, Pouplard-Barthelaix A, Benoit JP : Fate and biocompatibility of three types of microsheres implanted into the brain, J. Biomed Mat Res, 28, 1079-1085, 1994). Ce dernier résultat a été confirmé depuis par Kou JH, Emmett C, Shen P et al., Bioerosion and biocompatibility of poly(d,l-lactic-co-glycoljc acid) implants in brain, J Control Release, 43, 123-130, 1997.

Les microsphères biodégradables de l'invention ont de préférence un diamètre moyen de 48 ± 20 µm, de préférence 46 ± 7 µm. Elles contiennent 15 à 35 % en poids d'agent anticancéreux, de préférence de 19 à 27 % de 5-FU, de préférence encore 20% de 5-FU, et 65 à 85 % en poids de polymère.

Dans le cadre de la présente invention, les microsphères de PLAGA 50: 50 véhiculant du 5-FU sont particulièrement préférées.

In vitro, les microsphères de PLAGA 50: 50 véhiculant du 5-FU peuvent libérer du 5-FU pendant 21 jours. In vivo, implantées en sous-cutané chez le lapin, ces microsphères permettent d'obtenir une concentration plasmatique de 5-FU en plateau pendant 23 jours. Toujours in vivo et dans le cerveau de rongeurs, les cristaux de 5-FU sont visibles dans les microsphères au moins jusqu'au 19^{ème} jour. Chez le lapin, après implantation intra-cérébrale de microsphères de PLAGA-5-FU (7mg/kg de 5-FU), aucune trace de 5-FU n'est détectée dans le sérum, ce qui laisse suggérer un passage quasi nul de la drogue dans la circulation systémique.

Après implantation intra-cérébrale de microsphères de PLAGA-5-FU chez le rongeur, à une dose totale de 17 mg/kg de 5-FU, aucun signe de toxicité systémique, ni aucun signe de neurotoxicité clinique ou histologique n'a été observé. A la dose fractionnée de 24 Gy, l'association microsphères de 5-FU/radiothérapie cérébrale est parfaitement tolérée (Menei P, Vectorisation dans le SNC par implantation stéréotaxique de microsphères, Thèse d'Université en Sciences Pharmaceutiques, Université d'Angers, 1995). Enfin, ces microsphères implantées par stéréotaxie au sein d'un gliome malin développé chez le rat (gliome C6), diminuent significativement la mortalité (Menei P, Boisdron-Celle M, Croue A, Guy G, Benoit JP, Effect of stereotactic implantation of biodegradable 5-fluorouracil-loaded microspheres in normal and C6-glioma bearing rats, Neurosurgery, 39 : 117-124, 1996).

De façon avantageuse, les microsphères sont mises en suspension dans une solution stérile, la suspension étant injectée dans les parois du foyer opératoire après exérèse de la tumeur.

La solution stérile contient de préférence
- entre 1 et 1,5 %, de préférence 1,25 % poids/volume, d'un agent viscosifiant, par exemple la carboxyméthyl cellulose sodique,
- entre 0,5 et 1,5 %, de préférence 1 % d'un tensio-actif, par exemple le polysorbate 80®, et
- entre 3,5 et 4,5%, de préférence 4 %, d'un agent isotonisant, par exemple le mannitol.

Les microsphères sont de préférence mises en suspension de façon extemporanée juste avant injection. La suspension contient de préférence 3 ml de la solution stérile décrite précédemment et 700 à 800 mg de microsphères biodégradables.

Lorsque l'agent anticancéreux est le 5-FU, la dose totale de suspension injectée correspond à une quantité de 5-FU comprise entre 50 et 200 mg.

La radiothérapie est focalisée sur le volume tumoral, le volume irradié englobe la tumeur préopératoire avec une marge d'au moins deux centimètres dans toutes les directions, et une dose totale comprise entre 50 Gy et 60 Gy est appliquée.

La radiothérapie est amorcée de préférence entre les deuxième et septième jours consécutifs à l'opération. Une dose totale comprise entre 50 Gy et 60 Gy est étalée sur une durée comprise entre 4 et 8 semaines, par exemple à raison de 5 fractions par semaine.

La radiothérapie est de préférence effectuée avec une dose totale de 60 Gy pendant six semaines environ, de préférence encore à raison de cinq fractions par semaine pendant 6,5 semaines.

Après avoir injecté les microsphères juste après exérèse de la tumeur, une ou plusieurs nouvelles injections de microsphères peuvent être effectuées par stéréotaxie en cas de récurrence de la tumeur.

Les microsphères utilisées dans le cadre de l'invention peuvent être préparées par une technique d'émulsion-extraction, selon une variante du procédé décrit par Boisdron-Celle M, Menei P, Benoit JP : Preparation of biodegradable 5-fluorouracil-loaded microspheres, J Pharm Pharmacol, 47, 108-114, 1995.

La demande de brevet FR 2 693 905 porte sur un procédé de préparation de microsphères contenant l'hormone LHRH par émulsion-évaporation. Dans l'exemple 2 page 19, du PLAGA 75: 25 est dissous dans le dichlorométhane et l'hormone LHRH est dispersée dans un mélange dichlorométhane-éthanol. La dispersion d'hormone est ensuite ajoutée à la solution de polymère sous agitation. La phase organique est injectée à de l'eau à 19°C. Le dichlorométhane est évaporé sous agitation par bullage d'air. Après évaporation du solvant, les microsphères sont filtrées sous vide.

La présente invention concerne également un procédé de préparation des microsphères contenant un agent anticancéreux enrobées d'un polymère utilisées dans le cadre de l'invention. Les grandes étapes de ce procédé consistent à préparer une phase organique dans laquelle l'agent anticancéreux et le polymère sont dispersés dans un solvant organique. La phase organique et une phase aqueuse sont mises en émulsion, puis le solvant organique est extrait par addition d'eau. Enfin, la suspension de microsphères obtenue est filtrée.

Le procédé de l'invention est tout d'abord caractérisé en ce que l'agent anticancéreux est dispersé dans le solvant organique, sous vive agitation, avant l'ajout du polymère.

Selon les adaptations apportées au procédé de l'art antérieur, le principe actif est broyé dans un broyeur à bille planétaire. La taille des cristaux obtenus est comprise entre 15 et 50 µm. La taille des cristaux à encapsuler et leur dispersion sont en effet des critères essentiels pour maîtriser le taux d'encapsulation et la cinétique de libération in vitro.

Le principe actif est ensuite dispersé dans un solvant organique, de préférence le dichlorométhane, dans un tube à fond rond, sous agitation à l'aide d'une tige d'homogénéisation, avant l'ajout du polymère.

L'homogénéisation permet l'obtention d'une suspension homogène, l'atténuation des différences d'un lot de broyage à l'autre et la réduction de la taille des cristaux du principe actif.

La phase organique est préparée dans un solvant sans cosolvant. L'absence de cosolvant permet de ralentir la précipitation du polymère pendant la phase d'émulsion, si bien que les particules obtenues sont moins poreuses.

La dispersion de principe actif est transvasée dans un premier réacteur.

On ajoute le polymère en une proportion massique comprise entre 8 et 13 % de préférence égale à 11%. La phase organique obtenue est maintenue sous agitation constante à température ambiante pendant 2 à 4 heures puis 15 minutes environ à une température comprise entre 1 et 5°C, de préférence égale à 2°C. Une plus longue période d'agitation de la phase organique à température ambiante assure une totale solubilisation du polymère dans le solvant.

Dans un deuxième réacteur, on prépare la phase aqueuse en la maintenant de préférence à la même température que la phase organique, de préférence à 2°C. La diminution de la température de la phase aqueuse et de la phase organique provoque une augmentation de leur viscosité et une augmentation du taux d'encapsulation. La phase aqueuse est par exemple une solution aqueuse de PVA à 10 %.

On utilise deux réacteurs à doubles parois et un liquide réfrigérant circule en série dans les deux réacteurs. La température des phases organique et aqueuse est avantageusement identique, de préférence égale à environ 2°C, quand on mélange les deux phases. Une bonne maîtrise de la température conditionne en effet à la fois la taille des particules, la vitesse de dissolution du principe actif et la vitesse d'extraction du solvant.

La phase organique est transvasée du premier réacteur dans le second. La proportion volumique phase aqueuse / phase organique est comprise entre 80/3 et 120/3, de préférence égale à 100/3.

L'émulsion obtenue est agitée pendant au moins 3 minutes, de préférence pendant 3 à 6 minutes, de manière encore plus préférée pendant 5 minutes. Le choix de cette durée est directement corrélée à la cinétique de libération et notamment à l'effet « burst » sur 24-48 heures.

L'absence de cosolvant couplée à une durée d'émulsion suffisante, autorise la dissolution du principe actif en surface ou mal enrobé, si bien que les cinétiques de libération dans la phase initiale sont mieux maîtrisées.

On ajoute de l'eau à l'émulsion, en un rapport volumique émulsion / eau compris entre 1/4 et 1/2, de préférence égal à 1/3, pour extraire le solvant organique. La température de l'eau d'extraction est comprise entre 1 et 5°C, de préférence égale à 4°C.

Les étapes d'émulsion et d'extraction sont effectuées dans le même réacteur pour limiter la variabilité d'un lot à l'autre, et pour gagner du temps. La température de l'eau d'extraction est basse pour limiter une trop forte dissolution du principe actif.

La suspension de microsphères obtenue est mélangée pendant quelques minutes puis filtrée sous atmosphère inerte. Le travail sous atmosphère inerte permet de limiter les risques de contamination du produit.

Les microsphères, éventuellement obtenues selon le procédé décrit précédemment, sont avantageusement lyophilisées.

2 à 5 g de poudre (gâteau de filtration) de microsphères sont additionnés de 10 ml d'eau stérile. L'ensemble est congelé à -40 °C puis introduit dans le lyophilisateur. La lyophilisation dure 18 heures. En fin de manipulation, la température de dessiccation secondaire doit être maintenue en dessous de 10°C.

Les microsphères doivent être conservées à +4°C, même sèches.

La présente invention concerne également une suspension constituée d'une solution stérile contenant 1 à 1,5 % masse/volume d'un agent viscosifiant, 0,5 à 1,5 % d'un tensio-actif et 3,5 à 4,5 % d'un agent isotonisant, et des microsphères biodégradables libérant un agent anticancéreux enrobées d'un polymère décrites précédemment éventuellement obtenues selon le procédé décrit ci-dessus, la proportion des microsphères représentant 200 à 300 mg/ml de solution stérile, de préférence 230 à 270 mg/ml.

Ces microsphères sont constituées de préférence de 15 à 35% en poids d'agent anticancéreux et de 65 à 85% en poids de polymère.

Le polymère est avantageusement le poly(d,l-acide lactique-co-acide glycolique) contenant de préférence une quantité égale d'acide lactique et d'acide glycolique.

La solution stérile contient de préférence 1,25 % poids/volume de carboxyméthyl cellulose sodique, 1 % de polysorbate 80 et 4 % de mannitol.

### EXEMPLE 1

Des microsphères préparées par la technique d'émulsion-extraction de solvant, selon une variante du procédé décrit par Boisdron-Celle M, Menei P et Benoit JP (Preparation of biodegradable 5-fluorouracil-Ioaded microspheres, J Pharm Pharmacol, 47, 108-114, 1995).

### Broyage du 5-FU

Le 5-FU est broyé dans un broyeur à bille planétaire du type Pulvérisette 7 (Fritsch). 8,5 g de 5-FU sont introduits dans chaque jarre contenant 7 billes. Le broyage dure 10 minutes à vitesse 7. La poudre est récupérée sous hotte à flux laminaire. Les cristaux obtenus ont une taille comprise entre 15 et 50 µm, et se répartissent en deux fractions : une fraction fine (de granulométrie inférieure à 1 µm) et une fraction grosse (supérieure à 30 µm).

### Dispersion du 5-FU dans le solvant organique

Le 5-FU broyé est dispersé dans 45 ml de dichlorométhane sous agitation à l'aide d'un homogénéisateur du type ultra-turrax pendant 3 minutes à 13 500 tpm, dans un tube à fond rond.

### Préparation de la phase organique

La dispersion de 5-FU est transvasée dans un réacteur réfrigéré double paroi de 150 ml. On lui ajoute le PLAGA de telle sorte que la proportion PLAGA/dichlorométhane soit égale à 11 %. La phase organique est agitée avec une pale, à 450 tpm, pendant 4 heures à 20°C, puis 15 minutes à 2°C. Dans le réacteur la température est maintenue constante à 0,1°C près à l'aide d'un cryostat.

### Préparation de l'émulsion

On prépare 1500 ml d'une solution aqueuse autoclavée à 10 % de PVA maintenue à 2°C dans un réacteur de 6 litres réfrigéré à double paroi. La phase organique est ensuite transvasée dans ce réacteur par ouverture d'une vanne de fond du premier réacteur. La phase organique est déversée en 5 à 10 s sur la phase aqueuse agitée à l'aide d'une pale tournant à 375 tpm. La proportion volumique phase aqueuse / phase organique est égale à 100/3.

L'émulsion est agitée pendant 4 minutes et 45 s.

### Extraction

Lorsque l'émulsion est prête, on verse 4,5 l d'eau d'extraction à 4°C sur l'émulsion dans un rapport volumique émulsion/eau égal à 1/3. L'extraction dure 2 minutes.

### Filtration

La totalité du contenu du deuxième réacteur est transvasée par le fond dans une cuve en inox, puis mise sous pression d'azote. La suspension est filtrée sur un filtre dont le diamètre des pores est égal à 3 µm.

Après passage de la totalité de la suspension sur le filtre, le gâteau de filtration est lavé deux fois avec 3 litres d'eau stérile.

Le taux d'encapsulation de l'agent anticancéreux des microsphères obtenues est de 20 %. Après tamisage, une désorption du dichlorométhane est réalisée à l'étuve pendant 48 heures. Les microsphères sont ensuite conditionnées et stérilisées par γ-irradiation à 19 kGy. Un nouveau contrôle du taux d'encapsulation est réalisé après stérilisation. Un dosage des traces de solvant résiduel est ensuite effectué. On détecte avantageusement un taux résiduel de dichlorométhane de 0,5 %. On contrôle la stérilité et la cinétique de libération in vitro des microsphères obtenues.

Les microsphères obtenues ont une teneur en principe actif de 23 ± 3,5 %.

On réalise plusieurs lots en suivant le protocole décrit précédemment et on calcule une taille moyenne de particules de 48 ± 20 µm sur la population de l'ensemble des lots équivalant à 46 ± 7 µm (moyenne des moyennes des lots préparés).

Les microsphères obtenues ont une teneur en principe actif de 23 ± 3,5 % et une taille moyenne de 48 ± 20 µm.

### EXEMPLE 2

Des microsphères sont préparées par la technique d'émulsion-extraction de solvant de l'exemple 1,

### - Broyage du 5-FU

On procède comme dans l'exemple 1 en broyant 4 g de 5-FU

Les cristaux obtenus ont une taille comprise entre 15 et 50 µm, et se répartissent en deux fractions : une fraction fine (de granulométrie inférieure à 1 µm) et une fraction grosse (supérieure à 30 µm).

### - Dispersion du 5-FU dans le solvant organique

Le 5-FU broyé est dispersé dans 40 ml de dichlorométhane sous agitation à l'aide d'un homogénéisateur du type ultra-turrax pendant 3,5 minutes à 13 500 tpm, dans un tube à fond rond.
- La préparation de la phase organique et la préparation de l'émulsion sont réalisées comme dans l'exemple 1.
- L'extraction et la filtration sont réalisées comme dans l'exemple 1.

Caractéristique des microsphères obtenues.
Teneur en 5-FU : 22 %
Taille : 46 ± 7 µm
Effet burst à 24 h après radiostérilisation à 19 kGy : 40 ± 4 %.

### EXEMPLE 3

Une étude clinique pilote ouverte de phase I/II est réalisée avec les microsphères PLAGA 50 : 50 5-FU de l'exemple 1.

Les microsphères obtenues sont mises en suspension de façon extemporanée dans une solution contenant
- 1,25 % poids/volume de carboxyméthyl cellulose sodique (Cooper),
- 1 % de polysorbate 80,
- 4 % de mannitol, et
- une quantité suffisante d'eau pour préparation injectable pour obtenir un volume total de 3 ml.

La solution est préalablement stérilisée par autoclavage à 121°C pendant 20 minutes, puis par radiostérilisation par gamma-irradiation à une dose comprise entre 5 kGy et 25 kGy, de préférence 19 kGy.

La préparation de cette suspension est délicate car il faut éviter la formation de bulles. La suspension est injectée immédiatement après sa préparation, car les microsphères ont tendance à sédimenter dans le seringue et à la boucher.

La suspension de microsphères est implantée dans les parois du foyer opératoire, après exérèse macroscopique de la tumeur gliale, entre deux et trois centimètres de profondeur, tous les cm², à raison de 100 µl par injection.

L'injection est réalisée avec une seringue de 1 ml et un cathéter (Insyte® Vialon™) de 18 ga (1,3 x 45 mm) dont on retire le mandrin métallique pour ne garder que le cathéter en plastique à bout de mousse non biseauté pour injecter la suspension dans le tissu cérébral. On utilise autant de seringues de 1 ml que nécessaire. L'injection de la suspension avec l'aiguille biseautée expose au risque d'hématome et de reflux des microsphères. Le diamètre du cathéter doit être suffisamment petit pour ne pas traumatiser le tissu cérébral et suffisamment grand pour ne pas être bouché par la suspension de microsphères.

L'injection doit être réalisée très doucement et le cathéter doit rester quelques minutes en place avant d'être retiré pour éviter le reflux des microsphères. On applique sur le site d'injection un fragment de 1 cm² de compresse hématostatique résorbable (Surgical® ou Spongel®).

Les patients inclus dans l'étude sont âgés de 18 et 68 ans, sans antécédent néoplasique, avec un indice de Karnofsky supérieur à 60, ont une histoire clinique et une imagerie évoquant un glioblastome sustentoriel, ont subi une exérèse macroscopiquement complète, et leur examen histologique intra-opératoire (réalisé selon les critères de l'OMS : nécrose, prolifération vasculaire, pléomorphisme nucléaire et activité mitotique) confirme le diagnostic de glioblastome.

Les critères d'exclusion des patients sont les suivants : déficience métabolique, grossesse, autre pathologie cancéreuse antérieure.

Trois groupes étaient initialement prévus pour étudier les effets de doses croissantes de 5-fluoro-uracile : dans l'ordre chronologique, 70, 132 et 264 mg, le traitement du groupe suivant étant amorcé après avoir constaté la tolérance du traitement par le groupe en cours d'essai.

En raison de la survenue d'une toxicité neurologique de Grade II chez un patient ayant reçu 132 mg de 5-FU, et suivant les règles d'arrêt du protocole, l'escalade thérapeutique a été stoppée et les patients suivants ont reçu la même dose de 132 mg.

La radiothérapie externe conventionnelle (focalisée sur le volume tumoral apprécié sur l'IRM préopératoire avec une énergie de 10 MV) est amorcée entre les deuxième et septième jours consécutifs à l'opération. Une dose totale de 60 Gy en 33 fractions de 1,8 Gy, à raison de 5 fractions pendant 6,5 semaines, est appliquée. Le volume irradié englobe la tumeur préopératoire avec une marge d'au moins deux centimètres dans toutes les directions.

Les patients sont suivis sur le plan clinique et radiologique : un scanner à 72 heures pour confirmer l'exérèse macroscopiquement complète et une évaluation clinique sont effectués à J10, J20 et J30. Une IRM est réalisée à J10 et J30. Enfin, un dosage du 5-FU dans le sang et le LCR est réalisé à 72 heures, J10, J20 et J30. La toxicité (neurologique, hématologique, muqueuse et cardiologique) est évaluée en grades sur des critères dérivés de ceux de l'OMS. Passé un mois, les patients sont suivis cliniquement tous les deux mois et subissent une IRM tous les trois mois.

Une légère anémie post-opératoire, une hyperleucocytose et une légère lymphopénie ont été observées chez tous les patients.

L'étude pharmacologique a permis de confirmer la libération prolongée de 5-FU dans le liquide céphalo-rachidien (LCR) pendant plus de 30 jours, et un passage transitoire, à plus faible taux, de la molécule dans la circulation systémique. Des concentrations significatives de 5-FU sont encore présentes dans le LCR un mois après implantation.

Les profils de libération du 5-FU dans le LCR montrent un pic au dixième et au vingtième jour respectivement pour les doses de 70 et 132 mg. Le taux plasmatique de 5-FU n'était pas détectable à partir du dixième jour chez la moitié des patients.

La tolérance systémique est excellente chez tous les patients traités. Aucune modification de la chimie ou de la cellularité du LCR n'a été constatée. L'apparition d'un oedème cérébral durant la radiothérapie chez un patient traité avec 132 mg n'a pas permis de poursuivre l'escalade de la dose.

Huit patients, dont quatre hommes et quatre femmes, d'une moyenne d'âge égale à 48,5 ans et d'un indice de Karnofsky supérieur à 90, ont donc été inclus dans l'étude. Le premier groupe de trois a reçu une dose de 70 mg, et le deuxième groupe de cinq a reçu 132 mg.

Les résultats préliminaires sur la survie n'étaient pas interprétables sur le plan statistique en raison du petit nombre de patients. Ils étaient cependant très encourageants. A la dernière évaluation, dans le premier groupe traité (70 mg), les trois patients sont décédés à 61, 114 et 125 semaines. A noter que le patient décédé à 114 semaines l'a été de métastases pulmonaires du glioblastome. Dans le deuxième groupe traité (132 mg), trois patients sont décédés à 31, 59 et 82 semaines et 2 étaient toujours en rémission à 159 et 172 semaines, à la date d'écriture de ces résultats préliminaires.

La médiane de survie des patients est de 98 semaines (elle est de 50,6 semaines dans la littérature pour des patients répondant aux mêmes critères (Devaux BC, O'Fallon JR, Kelly PJ, Resection, biopsy, and survival in malignant glial neoplasms, J Neurosurg, 78: 767-775, 1993). Cinq patients sur huit, soit 62 % étaient vivants à 18 mois alors que dans la littérature, pour des patients répondant aux critères d'inclusion de cette étude, la survie à 18 mois est de 20 % (Devaux BC, O'Fallon JR, Kelly PJ, Resection, biopsy, and survival in malignant glial neoplasms, J Neurosurg, 78 : 767-775, 1993).

## Revendications

1. Procédé de préparation de microsphères biodégradables contenant un agent anticancéreux enrobé d'un polymère, par émulsion-extraction, qui consiste à disperser le polymère et l'agent anticancéreux dans un solvant organique, à mélanger la phase organique obtenue à une phase aqueuse pour obtenir une émulsion, à extraire le solvant organique par addition d'eau, puis à filtrer la suspension de microsphères obtenue, **caractérisé en ce que** l'agent anticancéreux est dispersé dans le solvant organique sous vive agitation avant l'ajout du polymère.

2. Procédé selon la revendication 1, **caractérisé en ce que** le solvant organique est le dichlorométhane.

3. Procédé selon la revendication 1 ou 2, **caractérisé en ce que** la phase aqueuse et la phase organique ont une température identique, de préférence égale à environ 2°C, quand elles sont mélangées.

4. Procédé selon l'une des revendications 1 à 3, **caractérisé en ce que** la phase organique contient 11 % de polymère.

5. Procédé selon l'une des revendications 1 à 4, **caractérisé en ce que** la proportion phase aqueuse/phase organique est égale à 100/3.

6. Procédé selon l'une des revendications 1 à 5, **caractérisé en ce que** l'émulsion constituée de la phase aqueuse et de la phase organique est mélangée pendant au moins 3 minutes.

7. Procédé selon l'une des revendications 1 à 6, **caractérisé en ce que** l'eau nécessaire pour extraire le solvant organique est ajoutée en une proportion telle que le rapport volumique émulsion/eau est égal à 1/3.

8. Procédé selon l'une des revendications 1 à 7, **caractérisé en ce que** l'eau nécessaire pour extraire le solvant organique a une température de 4°C.

9. Procédé selon l'une des revendications 1 à 8, **caractérisé en ce que** l'agent anticancéreux est broyé avant d'être dispersé dans le solvant organique, pour que la taille des cristaux de l'agent anticancéreux soit comprise entre 15 et 50 µm.

10. Procédé selon l'une des revendications 1 à 9, **caractérisé en ce que** après l'ajout de l'eau d'extraction, la suspension obtenue est filtrée sous atmosphère inerte.

11. Procédé selon l'une des revendications 1 à 10, **caractérisé en ce que** les microsphères sont lyophilisées.

12. Procédé selon l'une des revendications précédentes, **caractérisé en ce que** le polymère est choisi parmi l'éthylcellulose, le polystyrène, la poly(ε-caprolactone), le poly(d,l-acide lactique) et le poly(d,l-acide lactique-co-acide glycolique).

13. Procédé selon la revendication 12, **caractérisé en ce que** le polymère qui enrobe les microsphères est un poly(d,l-acide lactique-co-acide glycolique).

14. Procédé selon la revendication 13, **caractérisée en ce que** le polymère qui enrobe les microsphères est un poly(d.l-acide lactique-co-acide glycolique) contenant une quantité égale d'acide lactique et d'acide glycolique.

15. Suspension constituée d'une solution stérile contenant 1 à 1,5 % masse/volume d'un agent viscosifiant, 0,5 à 1,5 % d'un tensio-actif et 3,5 à 4,5 % d'un agent isotonisant, et de microsphères biodégradables susceptibles d'être obtenues par le procédé selon l'une quelconque des revendications 1 à 14, lesdites microsphères libérant un agent anticancéreux enrobées d'un polymère, la proportion des microsphères représentant 200 à 300 mg/ml de solution stérile, de préférence 230 à 270 mg/ml.

16. Suspension selon la revendication 15, **caractérisée en ce que** les microsphères sont constituées de 15 à 35% en poids d'agent anticancéreux et de 65 à 85% en poids de polymère.

17. Suspension selon la revendication 15 ou 16, **caractérisée en ce que** le polymère est le poly(d,i-acide lactique-co-acide glycolique) contenant de préférence une quantité égale d'acide lactique et d'acide glycolique.

18. Suspension selon l'une des revendications 15 à 17, **caractérisée en ce que** la solution stérile contient 1,25 % poids/volume de carboxyméthyl cellulose sodique, 1 % de polysorbate 80 et 4 % de mannitol.

19. Suspension selon l'une des revendications précédentes, **caractérisée en ce que** les microsphères ont un diamètre moyen de 48 ± 20 µm, de préférence 46 ± 7 µm.

20. Suspension selon l'une quelconque des revendications précédentes, **caractérisée en ce que** les microsphères contiennent 19 à 27 % de 5-FU, de préférence 20% de 5-FU.

21. Utilisation d'une suspension selon l'une quelconque des revendications 15 à 20 pour la fabrication d'un médicament destiné à être utilisé de façon simultanée, séparée ou étalée dans le temps avec une radiothérapie, pour le traitement du glioblastome, les microsphères étant destinées à être implantées, après exérèse de la tumeur gliale, dans les parois du foyer opératoire, à au moins deux centimètres de profondeur, de préférence entre 2 et 3 centimètres, au moins tous les cm², et le polymère qui les enrobe retardant la libération de l'agent anticancéreux et maintenant au cours du temps une concentration thérapeutiquement efficace dans l'espace parenchymateux en vue d'atteindre une durée de survie du patient ainsi traité au moins égale à environ 90 semaines, de préférence environ 130 semaines, de préférence encore environ 160 semaines.

22. Utilisation selon la revendication 21, **caractérisée en ce que** l'agent anticancéreux est hydrophile et/ou ne franchit pas la barrière hémato-encéphalique.

23. Utilisation selon la revendication 21 ou 22, **caractérisée en ce que** l'agent anticancéreux ne présente pas une neurotoxicité centrale.

24. Utilisation selon l'une des revendications précédentes, **caractérisée en ce que** l'agent anticancéreux est constitué d'un composé anticancéreux radiosensibilisant ou d'un mélange de composés anticancéreux contenant au moins un composé anticancéreux radiosensibilisant, le ou lesdits composés anticancéreux étant choisis parmi le 5-fluorouracile (5-FU), les platines, comme le carboplatine et le cisptatine, les taxanes, comme le docétaxel et le paclitaxel, la gemcitabine, le VP16, la mitomycine, l'idoxuridine, les inhibiteurs de la topoisomérase 1, comme l'irinotécan, le topotécan et les camptothécines, les nitroso-urées, comme le BCNU, l'ACNU ou le MCNU, le méthotrexate, la bléomycine, l'adriamycine, le cytoxan et la vincristine, les cytokines immunomodulatrices, comme l'IL2, l'IL6, l'IL12 et l'IL13 et les interférons.

25. Utilisation selon la revendication 24, **caractérisée en ce que** l'agent anticancéreux est le 5-fluorouracile.

26. Utilisation selon la revendication 25, **caractérisée en ce que** la concentration en 5-FU dans le liquide céphalo-rachidien, qui est le reflet de concentration dans l'espace parenchymateux, est comprise entre 3 et 20 ng/ml.

27. Utilisation selon l'une des revendications précédentes, **caractérisée en ce qu'**on adjoint à l'agent anticancéreux un composé neuroprotecteur choisi parmi les facteurs de croissance peptidiques, comme le NGF ou le BDNF.

28. Utilisation selon l'une des revendications précédentes, **caractérisée en ce que** le polymère enrobant les microsphères qui retarde la libération de l'agent anticancéreux maintient dans l'espace parenchymateux ladite concentration thérapeutiquement efficace pendant au moins trois semaines, de préférence au moins quatre semaines.

29. Utilisation selon la revendication 25, **caractérisée en ce que** la dose totale de 5-FU injectée est comprise entre 50 et 200 mg, de préférence 130 mg.

30. Utilisation selon l'une des revendications précédentes, **caractérisée en ce que** la radiothérapie est focalisée sur le volume tumoral, **en ce que** le volume irradié englobe la tumeur préopératoire avec une marge d'au moins deux centimètres dans toutes les directions et **en ce qu'**une dose totale comprise entre 50 Gy et 60 Gy est appliquée.

31. Utilisation selon l'une des revendications précédentes, **caractérisée en ce que** la radiothérapie est amorcée de préférence entre les deuxième et septième jours consécutifs à l'opération.

32. Utilisation selon l'une des revendications précédentes, **caractérisée en ce que** la radiothérapie est effectuée avec une dose totale de 60 Gy pendant six semaines environ.

33. Utilisation selon l'une quelconque des revendications précédentes, **caractérisée en ce que** la suspension de microsphères est injectée par stéréotaxie à une ou plusieurs reprises en cas de récurrence de la tumeur.

## Patentansprüche

1. Verfahren zur Herstellung von bioabbaubaren Mikrokugeln, die ein von einem Polymer umhülltes Antikrebsmittel enthalten, durch Emulsionsextraktion, die darin besteht, das Polymer und das Antikrebsmittel in einem organischen Lösungsmittel zu dispergieren, die erhaltene organische Phase mit einer wässrigen Phase zu mischen, um eine Emulsion zu erhalten, das organische Lösungsmittel durch Zugabe von Wasser zu extrahieren, dann die Suspension der erhaltenen Mikrokugeln zu filtrieren, **dadurch gekennzeichnet, dass** das Antikrebsmittel vor der Zugabe des Polymers unter schnellem Rühren in dem organischen Lösungsmittel dispergiert wird.

2. Verfahren nach Anspruch 1, **dadurch gekennzeichnet, dass** es sich bei dem organischen Lösungsmittel um Dichlormethan handelt.

3. Verfahren nach Anspruch 1 oder 2, **dadurch gekennzeichnet, dass** die wässrige Phase und die organische Phase eine identische Temperatur aufweisen, vorzugsweise gleich etwa 2 °C, wenn sie gemischt werden.

4. Verfahren nach einem der Ansprüche 1 bis 3, **dadurch gekennzeichnet, dass** die organische Phase 11 % Polymer enthält.

5. Verfahren nach einem der Ansprüche 1 bis 4, **dadurch gekennzeichnet, dass** das Verhältnis wässrige Phase/organische Phase gleich 100/3 ist.

6. Verfahren nach einem der Ansprüche 1 bis 5, **dadurch gekennzeichnet, dass** die Emulsion, die aus der wässrigen Phase und der organischen Phase besteht, mindestens während 3 Minuten gemischt wird.

7. Verfahren nach einem der Ansprüche 1 bis 6, **dadurch gekennzeichnet, dass** das Wasser, das erforderlich ist, um das organische Lösungsmittel zu extrahieren, in einem solchen Verhältnis zugesetzt wird, dass das Volumenverhältnis Emulsion/Wasser gleich 1/3 ist.

8. Verfahren nach einem der Ansprüche 1 bis 7, **dadurch gekennzeichnet, dass** das Wasser, das erforderlich ist, um das organische Lösungsmittel zu extrahieren, eine Temperatur von 4 °C aufweist.

9. Verfahren nach einem der Ansprüche 1 bis 8, **dadurch gekennzeichnet, dass** das Antikrebsmittel gemahlen wird, bevor es in dem organischen Lösungsmittel dispergiert wird, damit die Kristallgröße des Antikrebsmittels 15 bis 50 µm beträgt.

10. Verfahren nach einem der Ansprüche 1 bis 9, **dadurch gekennzeichnet, dass** nach der Zugabe des Wassers zur Extraktion die erhaltene Suspension unter Inertatmosphäre filtriert wird.

11. Verfahren nach einem der Ansprüche 1 bis 10, **dadurch gekennzeichnet, dass** die Mikrokugeln lyophilisiert werden.

12. Verfahren nach einem der vorangehenden Ansprüche, **dadurch gekennzeichnet, dass** das Polymer aus Ethylcellulose, Polystyrol, Poly(ε-caprolacton), Poly(d,l-milchsäure) und Poly(d,l-milchsäure-co-glycolsäure) ausgewählt ist.

13. Verfahren nach Anspruch 12, **dadurch gekennzeichnet, dass** das Polymer, welches die Mikrokugeln umhüllt, eine Poly(d,l-milchsäure-co-glycolsäure) ist.

14. Verfahren nach Anspruch 13, **dadurch gekennzeichnet, dass** das Polymer, welches die Mikrokugeln umhüllt, eine Poly(d,l-milchsäure-co-glycolsäure) ist, die eine gleiche Menge an Milchsäure und Glycolsäure enthält.

15. Suspension, zusammengesetzt aus einer sterilen Lösung, die 1 bis 1,5 % Gewicht/Volumen eines Viskositäts-erhöhenden Mittels, 0,5 bis 1,5 % eines Tensids und 3,5 bis 4,5 % eines isotonisierenden Mittels und bioabbaubare Mikrokugeln enthält, welche durch das Verfahren nach irgendeinem der Ansprüche 1 bis 14 erhältlich sind, wobei die Mikrokugeln ein von einem Polymer umhülltes Antikrebsmittel freisetzen, der Anteil der Mikrokugeln 200 bis 300 mg/ml sterile Lösung, vorzugsweise 230 bis 270 mg/ml beträgt.

16. Suspension nach Anspruch 15, **dadurch gekennzeichnet, dass** die Mikrokugeln aus 15 bis 35 Gew.-% Antikrebsmittel und aus 65 bis 85 Gew.-% Polymer zusammengesetzt sind.

17. Suspension nach Anspruch 15 oder 16, **dadurch gekennzeichnet, dass** das Polymer Poly(d,l-milchsäure-co-glycolsäure) ist, die vorzugsweise eine gleiche Menge an Milchsäure und an Glycolsäure enthält.

18. Suspension nach einem der Ansprüche 15 bis 17, **dadurch gekennzeichnet, dass** die sterile Lösung 1,25 % Gewicht/Volumen Natriumcarboxymethylcellulose, 1 % Polysorbat 80 und 4 % Mannit enthält.

19. Suspension nach einem der vorangehenden Ansprüche, **dadurch gekennzeichnet, dass** die Mikrokugeln einen mittleren Durchmesser von 48 ± 20 µm, vorzugsweise 46 ± 7 µm aufweisen.

20. Suspension nach irgendeinem der vorangehenden Ansprüche, **dadurch gekennzeichnet, dass** die Mikrokugeln 19 bis 27 % 5-FU, vorzugsweise 20 % 5-FU enthalten.

21. Verwendung einer Suspension nach irgendeinem der Ansprüche 15 bis 20 für die Herstellung eines Medikaments, das dazu bestimmt ist, auf gleichzeitige, getrennte oder zeitlich gestaffelte Weise mit einer Radiotherapie für die Behandlung von Glioblastom verwendet zu werden, wobei die Mikrokugeln dazu bestimmt sind, nach Exhärese des Gliatumors in den Wänden des Operationsgebietes bei mindestens 2 cm Tiefe, vorzugsweise zwischen 2 und 3 Zentimetern, mindestens alle cm² implantiert zu werden, wobei das Polymer, das sie umhüllt, die Freisetzung des Antikrebsmittels verzögert und im Lauf der Zeit eine therapeutisch wirksame Konzentration im parenchymatösen Raum aufrechterhält, mit dem Ziel, eine Überlebensdauer des so behandelten Patienten von mindestens gleich etwa 90 Wochen, vorzugsweise etwa 130 Wochen, noch bevorzugter etwa 160 Wochen, zu erreichen.

22. Verwendung nach Anspruch 21, **dadurch gekennzeichnet, dass** das Antikrebsmittel hydrophil ist und/oder nicht die Biut-Hirn-Schranke überquert.

23. Verwendung nach Anspruch 21 oder 22, **dadurch gekennzeichnet, dass** das Antikrebsmittel keine zentrale Neurotoxizität aufweist.

24. Verwendung nach einem der vorangehenden Ansprüche, **dadurch gekennzeichnet, dass** das Antikrebsmittel aus einer radiosensibilisierenden Antikrebs-Verbindung oder einer Mischung von Antikrebs-Verbindungen, die mindestens eine radiosensibilisierende Antikrebs-Verbindung enthalten, besteht, wobei das oder die Antikrebs-Verbindung(en) ausgewählt sind aus 5-Fluoruracil (5-FU), Platin-Verbindungen, wie Carboplatin und cis-Platin, Taxanen, wie Docetaxel und Paclitaxel, Gemcitabin VP16, Mitomycin, Idoxuridin, Inhibitoren der Topoisomerase 1, wie Irinotecan, Topotecan und den Camptothecinen, den Nitrosoharnstoffen, wie BCNU, ACNU oder MCNU, Methotrexat, Bleomycin, Adriamycin, Cytoxan und Vincristin, den immunomodulatorischen Cytokinen, wie IL2, IL6, IL12 und IL13 und den Interferonen.

25. Verwendung nach Anspruch 24, **dadurch gekennzeichnet, dass** das Antikrebsmittel 5-Fluoruracil ist.

26. Verwendung nach Anspruch 25, **dadurch gekennzeichnet, dass** die Konzentration des 5-FU im Gehirn-Rückenmark-Liquor, welche die Konzentration im parenchymatösen Raum widerspiegelt, bei 3 bis 20 ng/ml liegt.

27. Verwendung nach einem der vorangehenden Ansprüche, **dadurch gekennzeichnet, dass** man zu dem Antikrebsmittel eine Neuroschutz-Verbindung hinzufügt, die aus Peptid-Wachstumsfaktoren, wie NGF oder BDNF, ausgewählt ist.

28. Verwendung nach einem der vorangehenden Ansprüche, **dadurch gekennzeichnet, dass** das Polymer, das die Mikrokugeln umhüllt und die Freisetzung des Antikrebsmittels verzögert, die therapeutisch wirksame Konzentration im parenchymatösen Raum während mindestens 3 Wochen, vorzugsweise mindestens 4 Wochen, aufrecht erhält.

29. Verwendung nach Anspruch 25, **dadurch gekennzeichnet, dass** die Gesamt-Dosis an injiziertem 5-FU bei 50 bis 200 mg, vorzugsweise 130 mg, liegt.

30. Verwendung·nach einem der vorangehenden Ansprüche, **dadurch gekennzeichnet, dass** die Radiotherapie auf das Tumorvolumen fokussiert ist, dass das bestrahlte Volumen den voroperativen Tumor mit einem Randbereich von mindestens zwei Zentimetern in alle Richtungen umfasst und dass eine Gesamt-Dosis von 50 Gy bis 60 Gy angewendet wird.

31. Verwendung nach einem der vorangehenden Ansprüche, **dadurch gekennzeichnet, dass** die Radiotherapie vorzugsweise zwischen dem zweiten und dem siebten Tag, die auf die Operation folgen, eingeleitet wird.

32. Verwendung nach einem der vorangehenden Ansprüche, **dadurch gekennzeichnet, dass** die Radiotherapie mit einer Gesamt-Dosis von 60 Gy während etwa 6 Wochen bewirkt wird.

33. Verwendung nach einem der vorangehenden Ansprüche, **dadurch gekennzeichnet, dass** die Suspension von Mikrokugeln durch Stereotaxie mit einer oder mehreren Wiederholungen im Fall des Wiederauftretens des Tumors injiziert wird.

## Claims

1. Process for preparing biodegradable microspheres containing an anticancer agent, which are coated with a polymer, by emulsification-extraction, which consists in dispersing the polymer and the anticancer agent in an organic solvent, in mixing the organic phase obtained with an aqueous phase in order to obtain an emulsion, in extracting the organic solvent by addition of water and then in filtering the microsphere suspension obtained, **characterized in that** the anticancer agent is dispersed in the organic solvent with vigorous stirring before addition of the polymer.

2. Process according to Claim 1, **characterized in that** the organic solvent is dichloromethane.

3. Process according to Claim 1 or 2, **characterized in that** the aqueous phase and the organic phase have an identical temperature, preferably equal to about 2°C, when they are mixed together.

4. Process according to one of Claims 1 to 3, **characterized in that** the organic phase contains 11% of polymer.

5. Process according to one of Claims 1 to 4, **characterized in that** the aqueous phase/organic phase proportion is equal to 100/3.

6. Process according to one of Claims 1 to 5, **characterized in that** the emulsion consisting of the aqueous phase and the organic phase is mixed for at least 3 minutes.

7. Process according to one of Claims 1 to 6, **characterized in that** the water required to extract the organic solvent is added in a proportion such that the emulsion/water ratio by volume is equal to 1/3.

8. Process according to one of Claims 1 to 7, **characterized in that** the water required to extract the organic solvent has a temperature of 4°C.

9. Process according to one of Claims 1 to 8, **characterized in that** the anticancer agent is ground before being dispersed in the organic solvent, such that the size of the crystals of the anticancer agent is between 15 and 50 µm.

10. Process according to one of Claims 1 to 9, **characterized in that**, after adding the extraction water, the suspension obtained is filtered under an inert atmosphere.

11. Process according to one of Claims 1 to 10, **characterized in that** the microspheres are lyophilized.

12. Process according to one of the preceding claims, **characterized in that** the polymer is chosen from ethylcellulose, polystyrene, poly(ε-caprolacrtone), poly(d,l-lactic acid) and poly(d,l-lactic acid-co-glycolic acid).

13. Process according to Claim 12, **characterized in that** the polymer which coats the microspheres is a poly(d,l-lactic acid-co-glycolic acid).

14. Process according to Claim 13, **characterized in that** the polymer which coats the microspheres is a poly(d,l-lactic acid-co-glycolic acid) containing an equal amount of lactic acid and glycolic acid.

15. Suspension consisting of a sterile solution containing 1 to 1.5% mass/volume of a viscosity modifier, 0.5 to 1.5% of a surfactant and 3.5 to 4.5% of an isotonicity agent, and biodegradable microspheres which can be obtained using the process according to any one of Claims 1 to 14, said microspheres releasing an anticancer agent and being coated with a polymer, the proportion of microspheres representing 200 to 300 mg/ml of sterile solution, preferably 230 to 270 mg/ml.

16. Suspension according to Claim 15, **characterized in that** the microspheres consist of 15 to 35% by weight of anticancer agent and 65 to 85% by weight of polymer.

17. Suspension according to Claim 15 or 16, **characterized in that** the polymer is poly(d,1-lactic acid-co-glycolic acid) preferably containing an equal amount of lactic acid and glycolic acid.

18. Suspension according to one of Claims 15 to 17, **characterized in that** the sterile solution contains 1.25% weight/volume of sodium carboxymethylcellulose, 1% of polysorbate 80 and 4% of mannitol.

19. Suspension according to one of the preceding claims, **characterized in that** the microspheres have an average diameter of 48 ± 20 µm, preferably 46 ± 7 µm.

20. Suspension according to any one of the preceding claims, **characterized in that** the microspheres contain 19 to 27% of 5-FU, preferably 20% of 5-FU.

21. Use of a suspension according to any one of Claims 15 to 20, for the manufacture of a medicinal product intended to be used simultaneously or separately in time or spread out over time with a radiotherapy, for the treatment of glioblastoma, the microspheres being intended to be implanted into the walls of the operating locus, to a depth of at least two centimetres, preferably between 2 and 3 centimetres, at least every cm², after excision of the glial tumour, and the polymer which coats them delaying the release of the anticancer agent and maintaining a therapeutically effective concentration in the parenchymal space over time, in order to reach a survival time for the patient thus treated of at least about 90 weeks, preferably about 130 weeks, more preferably about 160 weeks.

22. Use according to Claim 21, **characterized in that** the anticancer agent is hydrophilic and/or does not cross the blood-brain barrier.

23. Use according to Claim 21 or 22, **characterized in that** the anticancer agent has no central neurotoxicity.

24. Use according to one of the preceding claims, **characterized in that** the anticancer agent consists of a radiosensitizing anticancer compound or a mixture of anticancer compounds containing at least one radiosensitizing anticancer compound, said anticancer compound(s) being chosen from 5-fluorouracil (5-FU), platinum agents, such as carboplatin and cisplatin, taxanes, such as docetaxel and paclitaxel, gemcitabine, VP16, mitomycin, idoxuridin, topoisomerase 1 inhibitors, such as irinotecan, topotecan and camptothecins, nitrosoureas, such as BCNU, ACNU or MCNU, methotrexate, bleomycin, adriamycin, cytoxan and vincristine, immunomodulatory cytokines, such as IL2, IL6, IL12 and IL13, and interferons.

25. Use according to Claim 24, **characterized in that** the anticancer agent is 5-fluorouracil.

26. Use according to Claim 25, **characterized in that** the concentration of 5-FU in the cerebrospinal fluid, which is a reflection of the concentration in the parenchymal space, is between 3 and 20 ng/ml.

27. Use according to one of the preceding claims, **characterized in that** a neuroprotective compound chosen from peptide growth factors, such as NGF or BDNF, is added to the anticancer agent.

28. Use according to one of the preceding claims, **characterized in that** the polymer coating the microspheres, which delays the release of the anticancer agent, maintains said therapeutically effective concentration in the parenchymal space for at least three weeks, preferably at least four weeks.

29. Use according to Claim 25, **characterized in that** the total dose of 5-FU injected is between 50 and 200 mg, preferably 130 mg.

30. Use according to one of the preceding claims, **characterized in that** the radiotherapy is focused on the tumour volume, **in that** the volume irradiated encompasses the preoperative tumour with a margin of at least two centimetres in all directions, and **in that** a total dose of between 50 Gy and 60 Gy is applied.

31. Use according to one of the preceding claims, **characterized in that** the radiotherapy is preferably started between the second and seventh days following the operation.

32. Use according to one of the preceding claims, **characterized in that** the radiotherapy is carried out with a total dose of 60 Gy for about six weeks.

33. Use according to any one of the preceding claims, **characterized in that** the suspension of microspheres is injected by stereotaxis one or more times in the event of recurrence of the tumour.
